# FASCICULE DE BREVET EUROPEEN

(11) **EP 2 794 096 B1**
(45) Date de publication et mention de la délivrance du brevet: **06.06.2018**
(21) Numéro de dépôt: 12821263.6
(22) Date de dépôt: 11.12.2012
(51) Int. Cl.: B01J 23/40, B01J 23/74, B01J 23/755, B01J 37/02, C10G 45/34, C10G 45/36, C10G 45/40

(54) **PROCEDE DE PREPARATION D'UN CATALYSEUR A BASE D'UN METAL DU GROUPE VIII PREPARE AVEC IMPREGNATION AU MOYEN D'AU MOINS UN ADDITIF ORGANIQUE**
HERSTELLUNGSVERFAHREN EINES GRUPPE VIII ENTHALTENDEN KATALYSATORS MITTELS IMPRAEGNIERUNG MIT EINEM ORGANISCHEN VERBINDUNG
PREPARATION PROCESS OF A GROUP VIII METAL COMPRISING CATALYST INVOLVING IMPREGNATION WITH AN ORGANIC COMPOUND

(30) Priorité: 21.12.2011 FR 1104003
(43) Date de publication de la demande: 29.10.2014
(73) Titulaire: IFP Energies nouvelles, 92500 Rueil-Malmaison (FR); Université Pierre et Marie Curie (Paris 6), 75252 Paris Cedex 05 (FR)
(72) Inventeur: BENTALEB, Faiza, F-75019 Paris (FR); DUBREUIL, Anne-Claire, F-69003 Lyon (FR); THOMAZEAU, Cecile, F-69008 Lyon (FR); MARCEAU, Eric, F-75013 Paris (FR); CHE, Michel, F-92340 Bourg la Reine (FR)
(86) Numéro de dépôt international: PCT/FR2012/000516
(87) Numéro de publication internationale: WO 2013/093231

(56) Documents cités:
- WO-A2-2010/097754
- JP-A- 7 136 523
- US-A- 5 945 459
- US-A- 6 136 868
- US-A1- 2004 204 506
- US-A1- 2005 113 251
- US-B1- 6 331 575

## Description

### Domaine de l'invention

Le domaine de l'invention est celui des procédés d'hydrogénation sélective. Le procédé d'hydrogénation sélective permet de transformer les composés polyinsaturés des coupes pétrolières par conversion des composés les plus insaturés vers les alcènes correspondants en évitant la saturation totale et donc la formation des alcanes correspondants.

L'objet de l'invention est de proposer un catalyseur à performances améliorées, notamment en termes de sélectivité tout en gardant des activités élevées, appliqué au procédé d'hydrogénation sélective des composés hydrocarbonés insaturés présents dans les coupes d'hydrocarbures, de préférence des coupes issues du vapocraquage. L'invention concerne plus particulièrement un procédé d'hydrogénation mettant en oeuvre ledit catalyseur.

### Art Antérieur

Les catalyseurs d'hydrogénation sélective sont généralement à base de métaux du groupe VIII du tableau périodique, de préférence le palladium ou le nickel. Le métal se présente sous la forme de particules métalliques nanométriques déposées sur un support qui peut être un oxyde réfractaire sous forme de billes, d'extrudés ou sous des formes présentant d'autres géométries. La teneur en métal, la taille des particules de métal et la répartition de la phase active dans le support font partie des critères qui ont une importance sur l'activité et la sélectivité des catalyseurs.

De nombreux travaux font état de l'utilisation de composés organiques comme additifs pour l'amélioration des performances catalytiques de catalyseurs métalliques. Cependant beaucoup de ces travaux concernent le domaine d'application des réactions Fischer Tropsch, dans lequel généralement des métaux tels que Cu, Ni ou Fe sont utilisés. Les brevets US6136868 et US6331575 enseignent respectivement l'introduction, lors de la préparation du catalyseur, de sucres de type mono- ou disaccharides, ou de polyols de formule générale CₙH₂ₙ₊₂Oₓ avec n un nombre entier compris entre 2 et 6, et x un entier compris entre 2 et 11, le sucrose étant particulièrement préféré. En ce qui concerne l'application en hydrogénation sélective, le brevet US3642658 reporte l'utilisation de composés organophosphorés pour l'amélioration des performances de catalyseurs d'hydrogénation sélective.

Le document US2005/113251 décrit un procédé d'hydrogénation sélective utilisant un catalyseur à base de rhodium et d'indium, ledit catalyseur étant préparé à l'aide de composés organiques oxygénés.

La présente invention décrit un type de catalyseur qui, de par son procédé spécifique de préparation va permettre d'obtenir un catalyseur plus sélectif en hydrogénation sélective tout en gardant une activité élevée. Le procédé de préparation comprend notamment la préparation d'une solution comprenant au moins un précurseur d'un métal du groupe VIII, avec au moins un additif, ledit additif et ledit métal étant introduits lors de la préparation de ladite solution dans un rapport molaire additif/ métal dudit métal du groupe VIII inférieur ou égal à 1,5. La présente invention a notamment pour objet un procédé d'hydrogénation sélective mettant en oeuvre le catalyseur.

### Résumé de l'invention

La présente invention a pour objet un procédé d'hydrogénation sélective d'une charge d'hydrocarbures polyinsaturés contenant au moins 2 atomes de carbone par molécule et ayant un point d'ébullition final inférieur ou égal à 250°C, par mise en contact de ladite charge avec au moins un catalyseur d'hydrogénation sélective dont la phase active est le nickel, déposée sur un support d'alumine, ladite mise en contact étant opérée à une température comprise entre 20 et 250°C, la vitesse horaire spatiale étant comprise entre 0,5 h-1 et 20 h-1, la pression étant comprise entre 0,3 MPa et 6,5 MPa, ledit catalyseur étant préparé selon un procédé comprenant :
a) au moins une étape de préparation d'une solution contenant au moins un précurseur dudit nickel et au moins un additif, ledit additif étant un composé organique présentant une à trois fonctions acides carboxyliques, ledit précurseur et ledit additif étant introduits lors de la préparation de la solution dans un rapport molaire additif/nickel inférieur ou égal à 1,5,
b) au moins une étape d'imprégnation de la solution préparée à l'étape a) sur ledit support,
c) au moins une étape de séchage du support imprégné issu de l'étape b),
d) au moins une étape de calcination du support séché issu de l'étape c) pour obtenir au moins ledit nickel sous forme oxyde.

La présente invention a notamment pour objet un procédé d'hydrogénation sélective mettant en oeuvre le catalyseur préparé selon l'invention.

De façon surprenante, il a été découvert qu'un catalyseur, dont la phase active est le nickel et préparé par imprégnation sur support d'alumine, d'une solution contenant au moins un précurseur dudit métal et au moins un additif, ledit additif et ledit métal étant introduits lors de la préparation de ladite solution dans un rapport molaire additif/ métal inférieur ou égal à 1,5, présente lorsqu'il est mis en oeuvre dans un procédé d'hydrogénation sélective, des performances catalytiques améliorées en termes de sélectivité tout en gardant des activités élevées.

### Description de l'invention

La présente invention concerne un procédé d'hydrogénation sélective d'une charge d'hydrocarbures polyinsaturés contenant au moins 2 atomes de carbone par molécule et ayant un point d'ébullition final inférieur ou égal à 250°C, par mise en contact de ladite charge avec au moins un catalyseur d'hydrogénation sélective dont la phase active est le nickel, déposée sur un support d'alumine, ladite mise en contact étant opérée à une température comprise entre 20 et 250°C, la vitesse horaire spatiale étant comprise entre 0,5 h-1 et 20 h-1, la pression étant comprise entre 0,3 MPa et 6,5 MPa, ledit catalyseur étant préparé selon un procédé comprenant :
a) au moins une étape de préparation d'une solution contenant au moins un précurseur dudit nickel et au moins un additif, ledit additif étant un composé organique présentant une à trois fonctions acides carboxyliques, ledit précurseur et ledit additif étant introduits lors de la préparation de la solution dans un rapport molaire additif/nickel inférieur ou égal à 1,5,
b) au moins une étape d'imprégnation de la solution préparée à l'étape a) sur ledit support,
c) au moins une étape de séchage du support imprégné issu de l'étape b),
d) au moins une étape de calcination du support séché issu de l'étape c) pour obtenir au moins ledit nickel sous forme oxyde.

Selon l'invention, la teneur en nickel dans ledit catalyseur est avantageusement comprise entre 0,01 et 50% poids de la masse du catalyseur.

L'additif est un composé organique présentant une à trois fonctions acides carboxyliques. De manière plus préférée, ledit additif est choisi parmi la glycine, l'acide aspartique, l'acide citrique et l'acide tartrique, de manière encore plus préférée, ledit additif est l'acide citrique.

Selon le procédé de préparation de l'invention, le rapport molaire additif/nickel dans la solution de l'étape a) est avantageusement compris entre 0,05 et 1,5, de préférence entre 0,1 et 1,5.

Le support utilisé est l'alumine.

Selon le procédé de préparation, l'étape c) de séchage est avantageusement réalisée à une température comprise entre 20 et 160°C pendant une durée comprise entre 1 et 24 heures.

Selon le procédé de préparation, l'étape d) de calcination est avantageusement réalisée à une température comprise entre 200 et 800°C pendant une durée comprise entre 1 et 6 heures.

Le procédé de préparation comprend avantageusement au moins une étape f) de traitement réducteur par mise en contact avec un gaz réducteur à une température supérieure ou égale à 100°C pendant une durée supérieure ou égale à 2 heures.

Dans une variante, le procédé de préparation comprend une étape e) de passivation du catalyseur par mise en contact avec au moins un composé soufré, après l'étape d) de calcination et avant l'étape f) de traitement réducteur.

Avantageusement, la charge d'hydrocarbures polyinsaturés mise en oeuvre dans le procédé d'hydrogénation sélective est choisie parmi la coupe C2 de vapocraquage, la coupe C3 de vapocraquage, la coupe C4 de vapocraquage, la coupe C5 de vapocraquage et les essences de vapocraquage et de manière plus préférée, ladite charge d'hydrocarbures polyinsaturés est une essence de vapocraquage.

### Description détaillée de l'invention

La présente invention a pour objet un procédé d'hydrogénation sélective d'une charge d'hydrocarbures polyinsaturés contenant au moins 2 atomes de carbone par molécule et ayant un point d'ébullition final inférieur ou égal à 250°C, par mise en contact de ladite charge avec au moins un catalyseur d'hydrogénation sélective dont la phase active est le nickel, déposée sur un support d'alumine, ladite mise en contact étant opérée à une température comprise entre 20 et 250°C, la vitesse horaire spatiale étant comprise entre 0,5 h-1 et 20 h-1, la pression étant comprise entre 0,3 MPa et 6,5 MPa, ledit catalyseur étant préparé selon un procédé comprenant :
a) au moins une étape de préparation d'une solution contenant au moins un précurseur dudit nickel et au moins un additif, ledit additif étant un composé organique présentant une à trois fonctions acides carboxyliques, ledit précurseur et ledit additif étant introduits lors de la préparation de la solution dans un rapport molaire additif/nickel inférieur ou égal à 1,5,
b) au moins une étape d'imprégnation de la solution préparée à l'étape a) sur ledit support,
c) au moins une étape de séchage du support imprégné issu de l'étape b),
d) au moins une étape de calcination du support séché issu de l'étape c) pour obtenir au moins ledit nickel sous forme oxyde.

Le catalyseur préparé selon l'invention comprend une phase métallique active déposée sur un support, ladite phase active est le nickel.

Le nickel se présente sous la forme de nanoparticules déposées sur ledit support. La teneur en nickel dans ledit catalyseur est avantageusement comprise entre 1 et 50 % poids de la masse du catalyseur, plus préférentiellement entre 5 et 40 % poids de la masse du catalyseur et encore plus préférentiellement entre 5 et 30% poids de la masse du catalyseur.

Le support sur lequel est déposée ladite phase active est une alumine.

Le volume poreux du support est généralement compris entre 0,1 cm³/g et 1,5 cm³/g, de préférence compris entre 0,5 cm³/g et 1 cm³/g.

La surface spécifique du support est généralement comprise entre 10 m²/g et 250 m²/g, de préférence entre 30 m²/g et 200 m²/g, de façon plus préférée entre 40 m²/g et 190 m²/g et de façon encore plus préférée entre 50 m²/g et 190 m²/g.

Ledit support poreux se présente avantageusement sous forme de billes, d'extrudés, de pastilles, ou d'agglomérats irréguliers et non sphériques dont la forme spécifique peut résulter d'une étape de concassage. De manière très avantageuse, ledit support se présente sous forme de billes ou d'extrudés et très préférentiellement sous forme de billes.

Selon l'invention, le support alumine peut comprendre des impuretés. Par exemple, le support peut comprendre des oxydes inorganiques tels que les oxydes de métaux des groupes IIA, IIIB, IVB, IIB, IIIA, IVA selon la classification CAS, de préférence la silice, le dioxyde de titane, le dioxyde de zirconium, l'oxyde de zinc, l'oxyde de magnésium et l'oxyde de calcium. La teneur en cation des colonnes IIA, IIIB, IVB, IIB, IIIA, IVA est de préférence comprise entre 0,01 et 30% poids, de préférence entre 0,01 et 10% poids, de façon encore plus préférée entre 0,03 et 1,5% poids.

### Étape a) du procédé de préparation

L'étape a) du procédé de préparation du catalyseur consiste en la préparation d'une solution contenant le précurseur du nickel et l'additif. Cette étape est réalisée dans tout solvant compatible avec la dissolution dudit précurseur de métal et dudit additif. De manière générale, l'étape a) est réalisée en phase aqueuse ou organique, de préférence en phase aqueuse. Lorsque le solvant utilisé est un solvant organique, il est avantageusement choisi parmi les solvants alcooliques tels que le méthanol, l'éthanol...De manière préférée, le solvant utilisé lors de l'étape a) est un solvant hydroalcoolique.

Lorsqu'il est introduit en solution organique, ledit précurseur du nickel est par exemple l'oxalate ou l'acétate. De manière préférée, ledit précurseur est introduit en solution aqueuse, par exemple sous forme de nitrate, de carbonate, d'acétate, de chlorure, d'oxalate, d'hydroxyde ou de tout autre dérivé inorganique soluble en solution aqueuse. On utilise avantageusement comme précurseur de nickel en phase aqueuse un précurseur choisi parmi le nitrate de nickel, le chlorure de nickel, le carbonate de nickel, l'acétate de nickel et l'hydroxyde de nickel, de manière préférée le nitrate de nickel.

Conformément au procédé de préparation, l'introduction de l'additif pour la préparation de la solution de l'étape a) est telle que le rapport molaire additif/ métal est inférieur ou égal à 1,5, de préférence compris entre 0,05 et 1,5, de manière préférée entre 0,1 et 1,5, de manière plus préférée entre 0,3 et 1,5, de manière encore plus préférée entre 0,5 et 1,3 et de manière très préférentielle de 0,7 à 1,3. Dans une variante préférée, l'introduction de l'additif pour la préparation de la solution de l'étape a) est telle que le rapport molaire additif/ métal est compris entre 0,8 et 1,3, de manière plus préférée entre 0,8 et 1,1 et de manière encore plus préférée entre 0,9 à 1,1.

Selon l'invention, l'additif est un composé organique présentant une à trois fonctions acides carboxyliques. De manière préférée, le composé organique est choisi parmi la glycine, l'acide aspartique, l'acide citrique et l'acide tartrique. De manière préférée, l'additif utilisé dans le procédé selon l'invention est l'acide citrique.

### Étape b) du procédé de préparation

L'étape b) du procédé de l'invention consiste en une étape d'imprégnation de la solution préparée à l'étape a) sur le support alumine. L'étape b) d'imprégnation peut être réalisée par toute méthode bien connue de l'homme du métier. En particulier l'étape b) peut être réalisée par imprégnation en excès ou par imprégnation à sec. De manière préférée, ladite étape b) est réalisée par imprégnation à sec, laquelle consiste à mettre en contact le support avec la solution aqueuse ou organique issue de l'étape a) avec un volume égal au volume poreux du support à imprégner. De manière très préférée, on réalise une seule étape d'imprégnation.

### Étape c) du procédé de préparation

L'étape c) de séchage, du support imprégné issu de l'étape b) est réalisée préférentiellement selon le procédé de préparation à une température comprise entre 20 et 160°C, de préférence entre 20 et 130°C. L'étape c) de séchage est préférentiellement réalisée pendant une durée comprise entre 1 et 24 heures, de préférence entre 1 et 20 heures.

### Étape d) du procédé de préparation

A l'issu de l'étape c) de séchage, une étape d) de calcination du support est réalisée à une température comprise entre 200 et 800°C, de préférence entre 250 et 600°C et de manière très préférée entre 300 et 600°C. Elle est préférentiellement réalisée pendant une durée comprise entre 1 et 6 heures. Le catalyseur obtenu à l'issue de ladite étape d) se trouve à l'état oxyde.

### Étape e) du procédé de préparation

Avantageusement, le procédé de préparation comprend une étape e) de passivation du catalyseur par un composé soufré, après l'étape d) de calcination et avant l'étape f) de traitement réducteur du catalyseur.

Le catalyseur est préparé selon un procédé comprenant avantageusement au moins une étape e) consistant à passiver ledit catalyseur par mise en contact avec au moins un composé soufré, ladite étape e) est effectuée après la mise en oeuvre de l'étape d) et avant la mise en oeuvre de l'étape f) de traitement réducteur.

Dans un mode de réalisation préféré, ladite étape e) est effectuée *ex-situ,* c'est-à-dire avant chargement du catalyseur dans l'unité réactionnelle d'hydrogénation sélective. Ladite étape e) est réalisée par la mise en oeuvre de méthodes connues de l'homme du métier et notamment, à titre d'exemple par la mise en oeuvre de l'une des méthodes décrites dans les documents de brevets EP 466.567(B1), US 5.153.163, FR 2.676.184 et WO 2004/098774. De manière préférée, ladite étape e) est effectuée par la mise en contact du catalyseur, obtenu à l'issue de la mise en oeuvre de l'étape d) avec au moins une solution comprenant au moins un réducteur organique et au moins un composé soufré. De manière très préférée, ladite étape e) est réalisée par imprégnation du catalyseur, obtenu à l'issue de la mise en oeuvre de l'étape d) avec ladite solution. Le réducteur organique présent dans ladite solution est par exemple choisi parmi l'acide formique, le formaldéhyde, l'acide acétique, l'éthanol, le méthanol, le formiate d'éthyl et le formiate de méthyle. Le composé soufré présent dans ladite solution est par exemple un composé de formule HO-R1-S-S-R2-OH (où R1 et R2 peuvent être tout type de radical organique) tel que le di-éthanol-di-sulfure (DEODS) ou encore un composé organique polysulfure de formule R-S(n)-R' où R et R' sont des radicaux organiques et n est compris entre 3 et 20, par exemple le dodecyl polysulfure. La quantité de soufre introduite est telle que le catalyseur passivé par le soufre comprenne de 0,2 à 2% poids de soufre. La quantité de réducteur organique introduite est telle que le catalyseur passivé comprenne entre 100 ppm (partie par million) et 50% poids dudit réducteur. Après introduction dudit composé soufré sur le catalyseur, ledit catalyseur est ensuite soumis à un traitement thermique réalisé à une température comprise entre 100 et 200°C pendant une durée comprise entre 30 minutes et 3 heures.

Le catalyseur comprenant ou non ladite étape e), se trouve, avant la mise en oeuvre du procédé d'hydrogénation sélective, au moins partiellement sous forme oxyde. Préalablement à la mise en oeuvre du procédé d'hydrogénation sélective, ledit catalyseur partiellement sous forme oxyde est avantageusement soumis à un traitement réducteur (étape f)).

### f) étape f) du procédé de préparation

Le procédé de préparation comprend avantageusement au moins une étape f) de traitement réducteur dont les modalités sont décrites ci-dessous.

Préalablement à son utilisation dans le réacteur catalytique et la mise en oeuvre du procédé d'hydrogénation sélective, le catalyseur est avantageusement soumis au moins à une étape f) de traitement réducteur, par mise en contact avec un gaz réducteur, par exemple avec de l'hydrogène, pur ou dilué, à haute température, typiquement supérieure ou égale à 100°C pendant une durée supérieure ou égale à 2 heures. Ce traitement permet d'activer ledit catalyseur et de former des particules de métal à l'état zéro valent. Ledit traitement réducteur peut être réalisé *in-situ* ou *ex-situ* c'est-à-dire avant le chargement du catalyseur dans le réacteur d'hydrogénation sélective. Ladite étape f) de traitement réducteur peut être mise en oeuvre sur le catalyseur ayant été soumis ou non à l'étape e) de passivation.

Dans une variante, lorsque le catalyseur n'a pas été soumis à ladite étape e) de passivation, ledit traitement réducteur est réalisé à une température comprise entre 100 et 600°C, de préférence entre 200 et 500°C, pendant une durée comprise entre 2 et 40 heures, de préférence entre 5 et 30 heures. La montée en température jusqu'à la température de réduction désirée est généralement lente, par exemple fixée entre 0,1 et 5°C/min. De manière préférée, ledit traitement réducteur est suivi d'au moins une étape de passivation par le soufre réalisée *in situ,* c'est-à-dire au sein du même réacteur que celui où est opérée la réaction d'hydrogénation sélective. Ladite étape de passivation est effectuée par injection d'au moins un composé soufré avant la mise en contact dudit catalyseur avec la charge d'hydrocarbures polyinsaturés à hydrogéner de manière à obtenir un catalyseur passivé au soufre dans lequel la teneur en soufre est comprise entre 0,1 et 2 % poids par rapport à la masse du catalyseur. Le composé soufré est par exemple choisi parmi les composés suivants : thiophène, thiophane, alkylmonosulfures tels que diméthylsulfure, diéthylsulfure, dipropylsulfure et propylméthylsulfure.

Dans une autre variante, lorsque le catalyseur a été soumis à ladite étape e) de passivation, ledit traitement réducteur est réalisé à une température comprise entre 100 et 400°C, de préférence entre 150 et 350°C, pendant une durée comprise entre 2 et 40 heures, de manière préférée entre 5 et 30 heures. La montée en température jusqu'à la température de réduction désirée est généralement lente, par exemple fixée entre 0,1 et 5°C/min.

### Procédé d'hydrogénation sélective

La présente invention concerne un procédé d'hydrogénation sélective d'une charge d'hydrocarbures polyinsaturés contenant au moins 2 atomes de carbone par molécule et ayant un point d'ébullition final inférieur ou égal à 250°C, par mise en contact de ladite charge avec au moins le catalyseur préparé selon le procédé de préparation.

La charge d'hydrocarbures polyinsaturés, traitée dans le procédé d'hydrogénation sélective, est préférentiellement choisie parmi la coupe C2 de vapocraquage, la coupe C3 de vapocraquage, la coupe C4 de vapocraquage, la coupe C5 de vapocraquage et les essences de vapocraquage encore appelées essence de pyrolyse. L'ensemble de ces coupes et les essences de vapocraquage contiennent au moins 2 atomes de carbone par molécule et présente un point d'ébullition final inférieur ou égal à 250°C. Plus précisément, lesdits hydrocarbures polyinsaturés présents dans ladite charge traitée sont en particulier des composés comportant au moins une fonction acétylénique (c'est-à-dire au moins une triple liaison) et/ou au moins une fonction diénique (c'est-à-dire au moins deux double liaisons). En particulier, ladite charge d'hydrocarbures polyinsaturés peut comprendre au moins un type de composé contenant à la fois une fonction acétylène et une fonction diénique par molécule.

Les procédés de conversion des hydrocarbures tels que le procédé de vapocraquage, dont est préférentiellement issue ladite charge hydrocarbonée à traiter selon le procédé d'hydrogénation sélective utilisant le catalyseur préparé selon le procédé de l'invention, sont opérés à haute température et produisent une grande variété de molécules mono-insaturées telles que l'éthylène, le propylène, les butènes linéaires, l'isobutène, les pentènes ainsi que des molécules mono-insaturées contenant jusqu'à environ 15 atomes de carbone. En parallèle sont également formés des composés polyinsaturés ayant plusieurs doubles liaisons et/ou au moins une triple liaison, en particulier de l'acétylène, du propadiène et du méthylacétylène (ou propyne), du 1,2 et 1,3 butadiène, du butyne, du vinylacétylène et de l'éthylacétylène, du pentadiène ainsi que d'autres composés polyinsaturés présents dans les essences de vapocraquage, en particulier des composés styréniques et indéniques.

La coupe C2 de vapocraquage, avantageusement utilisée pour la mise en oeuvre du procédé d'hydrogénation sélective selon l'invention, présente par exemple la composition suivante : 90% poids d'éthylène, de l'ordre de 0,3 à 2% poids d'acétylène, le reste étant essentiellement de l'éthane. Dans certaines coupes C2 de vapocraquage, entre 0,1 et 1 % poids de composés en C3 peut aussi être présent. La coupe C3 de vapocraquage, avantageusement utilisée pour la mise en oeuvre du procédé d'hydrogénation sélective selon l'invention, présente par exemple la composition moyenne suivante : de l'ordre de 90% poids de propylène, de l'ordre de 3 à 8% poids de propadiène et de méthylacétylène, le reste étant essentiellement du propane. Dans certaines coupes C3, entre 0,1 et 2% poids de composés en C2 et de composés en C4 peut aussi être présent. La coupe C4 de vapocraquage, avantageusement utilisée pour la mise en oeuvre du procédé d'hydrogénation sélective selon l'invention, présente par exemple la composition massique moyenne suivante : 1 % poids de butane, 46,5% poids de butène, 51% poids de butadiène, 1,3% poids de VinylAcetylène (VAC) et 0,2% poids de butyne. Dans certaines coupes C4, entre 0,1 et 2% poids de composés en C3 et de composés en C5 peut aussi être présent. La coupe C5 de vapocraquage, avantageusement utilisée pour la mise en oeuvre du procédé d'hydrogénation sélective selon l'invention, présente par exemple la composition suivante : 21% poids de pentanes, 45% poids de pentènes, 34% poids de pentadiènes.

L'essence de vapocraquage ou essence de pyrolyse, avantageusement utilisée pour la mise en oeuvre du procédé d'hydrogénation sélective selon l'invention, correspond à une coupe hydrocarbonée dont la température d'ébullition est généralement comprise entre 0°C et 250°C, de préférence entre 10°C et 220°C. Les hydrocarbures polyinsaturés présents dans ladite essence de vapocraquage sont en particulier des composés dioléfiniques (butadiène, isoprène, cyclopentadiène), des composés styréniques (styrène, alpha-méthylstyrène) et des composés indéniques (indène). L'essence de vapocraquage comprend généralement la coupe C5-C12 avec des traces de C3, C4, C13, C14, C15 (par exemple entre 0,1 et 3% poids pour chacune de ces coupes). Par exemple, l'essence de vapocraquage peut avantageusement présenter la répartition suivante, selon les fonctions chimiques présentes dans les composés de la charge hydrocarbonée (en % poids) :
Paraffines + naphtènes : 10-25
Aromatiques : 50-70
Mono-oléfines : 5-20
Dioléfines : 10-25
Alkénylaromatiques (par exemple le styrène) : 2-10
Soufre 5 - 500 ppm

De manière préférée, la charge d'hydrocarbures polyinsaturés traitée conformément au procédé d'hydrogénation sélective utilisant le catalyseur préparé selon le procédé est une essence de vapocraquage.

Le procédé d'hydrogénation sélective selon l'invention vise à éliminer lesdits hydrocarbures polyinsaturés présents dans ladite charge à hydrogéner en procédant à la conversion desdits hydrocarbures polyinsaturés vers les alcènes correspondants en évitant la saturation totale desdits hydrocarbures de manière à éviter la formation des alcanes correspondants. Par exemple, lorsque ladite charge est une essence de vapocraquage, le procédé d'hydrogénation sélective selon l'invention vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants.

La mise en oeuvre technologique du procédé d'hydrogénation sélective selon l'invention est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs ré-injection(s) de l'effluent, issu dudit réacteur où se produit la réaction d'hydrogénation sélective, en divers points du réacteur, situés entre l'entrée et la sortie du réacteur. La mise en oeuvre technologique du procédé d'hydrogénation sélective selon l'invention peut également être avantageusement réalisée par l'implantation d'au moins dudit catalyseur préparé selon le procédé de l'invention supporté dans une colonne de distillation réactive ou dans des réacteurs - échangeurs. Le flux d'hydrogène peut être introduit en même temps que la charge à hydrogéner et/ou en un point différent du réacteur.

Suivant la nature des charges à hydrogéner, le procédé d'hydrogénation sélective selon l'invention est réalisé soit en phase liquide soit en phase gazeuse. Dans le cas où la charge à traiter est une coupe C2 de vapocraquage, le procédé d'hydrogénation sélective selon l'invention est mis en oeuvre en phase gazeuse, avec une pression totale généralement comprise entre 1 et 5 MPa, une température comprise entre 20 et 250°C, de préférence entre 35 et 150°C, un rapport molaire hydrogène /(hydrocarbures polyinsaturés à hydrogéner) compris entre 0,8 et 200, de préférence entre 0,9 et 120. La vitesse spatiale horaire (définie comme le rapport du débit volumique de charge sur le volume de catalyseur) est comprise entre 1000 et 20000 h⁻¹, de préférence entre 4000 et 12000 h⁻¹.

Dans le cas où la charge est une coupe C3 de vapocraquage, une coupe C4 de vapocraquage, une coupe C5 de vapocraquage ou une essence de vapocraquage, le procédé d'hydrogénation sélective selon l'invention est mis en oeuvre en phase liquide dans les conditions opératoires suivantes : une pression totale comprise entre 0,3 et 5 MPa, une température comprise entre 20 et 200°C et un rapport molaire hydrogène /(hydrocarbures polyinsaturés à hydrogéner) compris entre 0,1 et 4, de préférence entre 1 et 2. La vitesse spatiale horaire (définie comme le rapport du débit volumique de charge sur le volume de catalyseur), établie dans ces conditions, est généralement comprise entre 0,2 et 100 h⁻¹, de préférence entre 5 et 80 h⁻¹ et de manière encore plus préférée entre 10 et 50 h⁻¹.

Dans le cas préféré où la charge d'hydrocarbures polyinsaturés est une essence de vapocraquage, l'hydrogénation sélective est effectuée en présence du catalyseur préparé selon le procédé de l'invention sous pression, en phase liquide, en présence d'une quantité d'hydrogène en faible excès par rapport à la valeur stoechiométrique permettant l'hydrogénation sélective des hydrocarbures polyinsaturés présents dans la charge, c'est-à-dire un excès compris avantageusement entre 5 et 30%. Pour l'hydrogénation sélective d'une essence de vapocraquage, le procédé de l'invention est mis en oeuvre à une température comprise entre 20 et 250°C, sous une pression totale généralement suffisante pour maintenir au moins 80% poids de la charge à traiter en phase liquide à l'entrée de l'unité réactionnelle. Ladite pression est préférentiellement comprise entre 0,3 et 6,5 MPa, plus préférentiellement comprise entre 1 et 5 MPa, de manière plus préférée entre 1 et 4 MPa. La vitesse spatiale horaire (définie comme le rapport du débit volumique de charge sur le volume de catalyseur), établie dans ces conditions, est généralement comprise entre 0,5 et 20 h⁻¹, de préférence entre 1 et 10 h⁻¹ et de manière encore plus préférée entre 2 et 10 h⁻¹.

L'invention est illustrée par les exemples qui suivent sans pour autant en limiter la portée.

### Exemples

Les catalyseurs des exemples 1, 2 et 3 sont préparés à isoteneur en élément nickel. Le support utilisé pour la préparation de chacun de ces catalyseurs est un support formé de billes d'alumine delta ayant un volume poreux de 0,67 cm³/g et une surface BET égale à 140 m²/g.

### Exemple 1 (comparatif) : Préparation d'un catalyseur supporté A de formule Ni/Al₂O₃ par imprégnation de nitrate de nickel sans additif

Une solution aqueuse de nitrate de nickel est préparée à 25°C par dilution de 24,71 g de nitrate de nickel Ni(NO₃)₂, 6H₂O dans de l'eau déminéralisée à un volume qui correspond au volume poreux dudit support d'alumine. Cette solution est ensuite imprégnée sur 50 g dudit support d'alumine. Le solide est ensuite séché à 25°C sous air pendant une nuit, puis calciné sous air à 500 °C pendant 2 heures.

Le catalyseur oxyde A ainsi préparé contient 10% poids de l'élément nickel supporté sur alumine.

### Exemple 2 (invention) : Préparation d'un catalyseur supporté B de formule Ni/Al₂O₃ par imprégnation d'une solution contenant du nickel et de l'acide citrique dans un rapport molaire acide citrique/nickel égal à 0,1

Une solution aqueuse est préparée par dissolution de 24,71 g de nitrate de nickel Ni(NO₃)₂, 6H₂O et de 1,63 g d'acide citrique dans de l'eau déminéralisée à un volume qui correspond au volume poreux dudit support d'alumine et dans un rapport molaire acide citrique/nickel égal à 0,1. Cette solution est ensuite imprégnée à sec sur ledit support d'alumine. Le solide B ainsi obtenu est ensuite séché à 25°C sous air pendant une nuit, puis calciné sous air à 500°C pendant 2 heures.

Le catalyseur oxyde B ainsi préparé contient 10% poids de l'élément nickel supporté sur alumine.

### Exemple 3 (invention) : Préparation d'un catalyseur supporté C de formule Ni/Al₂O₃ par imprégnation d'une solution contenant du nickel et de l'acide citrique dans un rapport molaire acide citrique/nickel égal à 1

Une solution aqueuse est préparée par dissolution de 24,71 g de nitrate de nickel Ni(NO₃)₂, 6H₂O et de 16,31 g d'acide citrique dans de l'eau déminéralisée à un volume qui correspond au volume poreux dudit support d'alumine et dans une rapport molaire acide citrique/nickel égal à 1. Cette solution est ensuite imprégnée à sec sur ledit support d'alumine. Le solide C ainsi obtenu est ensuite séché à 25°C sous air pendant une nuit, puis calciné sous air à 500°C pendant 2 heures.

Le catalyseur oxyde C ainsi préparé contient 10% poids de l'élément nickel supporté sur alumine.

### Exemple 4 : Évaluation des propriétés catalytiques des catalyseurs A. B et C en hydrogénation sélective d'un mélange comprenant du styrène et de l'isoprène

Chacun des catalyseurs A, B et C avant d'être successivement testé en hydrogénation sélective d'un mélange styrène - isoprène, est réduit *ex situ* sous un flux de 1 litre d'hydrogène par heure et par gramme de catalyseur avec une montée en température de 1°C/min et un palier à 400°C pendant 16 heures.

La réaction d'hydrogénation sélective est opérée dans un réacteur fermé parfaitement agité, ledit réacteur étant chargé avec 4 millilitres de billes de catalyseur ayant subi la réduction mentionnée ci-dessus, lesquelles ont été transférées à l'abri de l'air dans le réacteur.

La composition de la charge à hydrogéner sélectivement est la suivante : 8% poids styrène, 8% poids isoprène.

La réaction d'hydrogénation sélective est réalisée en phase liquide. Le test est réalisé sous une pression constante de 3,5 MPa d'hydrogène et à une température de 45 °C. La consommation d'hydrogène est suivie au cours du temps par la perte de pression dans une bouteille réservoir située en amont du réacteur.

Le tableau 1 présente les résultats de sélectivité vers la formation d'oléfines mesurée à 90% de conversion de l'isoprène.

Les activités catalytiques, reportées dans le tableau 1, sont exprimées en moles de H₂ consommées par minute et par gramme de catalyseur.

**Tableau 1: Sélectivité et activités catalytiques mesurées en hydrogénation d'un mélange styrène-isoprène**

| Catalyseur | Sélectivité | Activité* |
|---|---|---|
| Catalyseur A (comparatif) | 88,3 | 6.10⁻² |
| Catalyseur B (invention) | 89 | 5,8. 10⁻² |
| Catalyseur C (invention) | 95,8 | 6.10⁻² |

| | | |
|---|---|---|
| *en (moles H₂)/[min×(gramme de catalyseur)] | | |

Les résultats figurant dans le tableau 1 démontrent que les catalyseurs B et C sont plus sélectifs que le catalyseur A préparé en l'absence d'acide citrique tout en gardant une activité élevée équivalente à celle du catalyseur A. Le procédé de préparation confère donc au catalyseur obtenu des caractéristiques permettant d'obtenir de meilleures propriétés en hydrogénation sélective.

Sans être lié par une quelconque théorie, il semble que les propriétés améliorées du catalyseur proviennent d'une dispersion plus efficace de la phase active obtenue grâce au procédé spécifique de préparation.

## Revendications

1. Procédé d'hydrogénation sélective d'une charge d'hydrocarbures polyinsaturés contenant au moins 2 atomes de carbone par molécule et ayant un point d'ébullition final inférieur ou égal à 250°C, par mise en contact de ladite charge avec au moins un catalyseur d'hydrogénation sélective dont la phase active est le nickel, déposée sur un support d'alumine, ladite mise en contact étant opérée à une température comprise entre 20 et 250°C, la vitesse horaire spatiale étant comprise entre 0,5 h-1 et 20 h-1, la pression étant comprise entre 0,3 MPa et 6,5 MPa, ledit catalyseur étant préparé selon un procédé comprenant :
a) au moins une étape de préparation d'une solution contenant au moins un précurseur dudit nickel et au moins un additif, ledit additif étant un composé organique présentant une à trois fonctions acides carboxyliques, ledit précurseur et ledit additif étant introduits lors de la préparation de la solution dans un rapport molaire additif/nickel inférieur ou égal à 1,5,
b) au moins une étape d'imprégnation de la solution préparée à l'étape a) sur ledit support,
c) au moins une étape de séchage du support imprégné issu de l'étape b),
d) au moins une étape de calcination du support séché issu de l'étape c) pour obtenir au moins ledit nickel sous forme oxyde.

2. Procédé selon la revendication précédente tel que la teneur en nickel dans ledit catalyseur est comprise entre 0,01 et 50% poids de la masse du catalyseur.

3. Procédé selon la revendication 1 ou 2 tel que ledit additif est choisi parmi la glycine, l'acide aspartique, l'acide citrique et l'acide tartrique.

4. Procédé selon la revendication 3 tel que ledit additif est l'acide citrique.

5. Procédé selon l'une des revendications précédentes tel que le rapport molaire additif/nickel dans la solution de l'étape a) est compris entre 0,05 et 1,5.

6. Procédé selon la revendication 5 tel que le rapport molaire additif/nickel dans la solution de l'étape a) est compris entre 0,1 et 1,5.

7. Procédé selon l'une des revendications précédentes tel que l'étape c) de séchage est réalisée à une température comprise entre 20 et 160°C pendant une durée comprise entre 1 et 24 heures.

8. Procédé selon l'une des revendications précédentes tel que l'étape d) de calcination est réalisée à une température comprise entre 200 et 800°C pendant une durée comprise entre 1 et 6 heures.

9. Procédé selon l'une des revendications précédentes comprenant au moins une étape f) de traitement réducteur par mise en contact avec un gaz réducteur à une température supérieure ou égale à 100°C pendant une durée supérieure ou égale à 2 heures

10. Procédé selon l'une des revendications 1 à 9 comprenant une étape e) de passivation du catalyseur par mise en contact avec au moins un composé soufré, après l'étape d) de calcination et avant l'étape f) de traitement réducteur.

11. Procédé selon les revendications 1 à 10 tel que ladite charge d'hydrocarbures polyinsaturés est choisie parmi la coupe C2 de vapocraquage, la coupe C3 de vapocraquage, la coupe C4 de vapocraquage, la coupe C5 de vapocraquage et les essences de vapocraquage.

12. Procédé selon les revendications 1 à 11 tel que ladite charge d'hydrocarbures polyinsaturés est une essence de vapocraquage.

## Patentansprüche

1. Verfahren zur selektiven Hydrierung einer Charge von mehrfach ungesättigten Kohlenwasserstoffen, die mindestens 2 Kohlenstoffatome pro Molekül aufweist und einen Endsiedepunkt kleiner oder gleich 250 °C hat, durch Kontaktierung der Charge mit mindestens einem Katalysator zur selektiven Hydrierung, dessen Aktivphase Nickel ist, die auf einen Aluminiumoxidträger aufgebracht wird, wobei die Kontaktierung bei einer Temperatur zwischen 20 und 250 °C erfolgt, wobei die stündliche Raumgeschwindigkeit zwischen 0,5 h - 1 und 20 h-1 beträgt, wobei der Druck zwischen 0,3 MPa und 6,5 MPa beträgt, wobei der Katalysator nach einem Verfahren hergestellt wird, umfassend:
a) mindestens einen Schritt der Herstellung einer Lösung, die mindestens einen Nickel-Vorläufer und mindestens einen Zusatzstoff enthält, wobei der Zusatzstoff eine organische Verbindung ist, die eine bis drei Carboxylsäurefunktionen aufweist, wobei der Vorläufer und der Zusatzstoff bei der Herstellung der Lösung in einem Molverhältnis Zusatzstoff/Nickel kleiner oder gleich 1,5 eingeführt werden,
b) mindestens einen Schritt des Imprägnierens der in Schritt a) hergestellten Lösung auf dem Träger,
c) mindestens einen Schritt des Trocknens des imprägnierten Trägers aus Schritt b),
d) mindestens einen Schritt des Kalzinierens des getrockneten Trägers aus Schritt c), um mindestens das Nickel in Oxidform zu erhalten.

2. Verfahren nach dem vorhergehenden Anspruch, wobei der Nickelgehalt in dem Katalysator zwischen 0,01 und 50 Gew.-% der Masse des Katalysators beträgt.

3. Verfahren nach Anspruch 1 oder 2, wobei der Zusatzstoff unter Glycin, Asparaginsäure, Zitronensäure und Weinsäure ausgewählt ist.

4. Verfahren nach Anspruch 3, wobei der Zusatzstoff Zitronensäure ist.

5. Verfahren nach einem der vorhergehenden Ansprüche, wobei das Molverhältnis Zusatzstoff/Nickel in der Lösung des Schrittes a) zwischen 0,05 und 1,5 beträgt.

6. Verfahren nach Anspruch 5, wobei das Molverhältnis Zusatzstoff/Nickel in der Lösung des Schrittes a) zwischen 0,1 und 1,5 beträgt.

7. Verfahren nach einem der vorhergehenden Ansprüche, wobei der Schritt c) des Trocknens bei einer Temperatur zwischen 20 und 160 °C während einer Dauer zwischen 1 und 24 Stunden durchgeführt wird.

8. Verfahren nach einem der vorhergehenden Ansprüche, wobei der Schritt d) des Kalzinierens bei einer Temperatur zwischen 200 und 800 °C während einer Dauer zwischen 1 und 6 Stunden durchgeführt wird.

9. Verfahren nach einem der vorhergehenden Ansprüche, umfassend mindestens einen Schritt f) der Reduktionsbehandlung durch Kontaktnahme mit einem Reduktionsgas bei einer Temperatur größer oder gleich 100 °C während einer Dauer größer oder gleich 2 Stunden.

10. Verfahren nach einem der Ansprüche 1 bis 9, umfassend einen Schritt e) der Passivierung des Katalysators durch Kontaktierung mit mindestens einer schwefelhaltigen Verbindung nach dem Schritt d) des Kalzinierens und vor dem Schritt f) der Reduktionsbehandlung.

11. Verfahren nach den Ansprüchen 1 bis 10, wobei die Charge von mehrfach ungesättigten Kohlenwasserstoffen unter der Dampfcrackfraktion C2, der Dampfcrackfraktion C3, der Dampfcrackfraktion C4, der Dampfcrackfraktion C5 und den Dampfcrackbenzinen ausgewählt ist.

12. Verfahren nach den Ansprüchen 1 bis 11, wobei die Charge von mehrfach ungesättigten Kohlenwasserstoffen ein Dampfcrackbenzin ist.

## Claims

1. A process for the selective hydrogenation of a polyunsaturated hydrocarbon feed containing at least 2 carbon atoms per molecule and with an end point of 250°C or less, by bringing said feed into contact with at least a selective hydrogenation catalyst wherein the active phase is nickel, deposited on an alumina support, said bringing into contact is carried out at a temperature in the range 20°C to 250°C, the hourly space velocity being in the range 0.5 h⁻¹ to 20 h⁻¹, the pressure being in the range 0.3 MPa to 6.5 MPa, said catalyst being prepared by a process comprising:
a) at least one step for preparing a solution containing at least one precursor of nickel and at least one additive, said additive is an organic compound having one to three carboxylic acid functions, said precursor and said additive being introduced during the preparation of the solution in an additive/nickel molar ratio of 1.5 or less;
b) at least one step for impregnating the solution prepared in step a) onto said support;
c) at least one step for drying the impregnated support obtained from step b);
d) at least one step for calcining the dried support from step c) in order to obtain at least said nickel in the oxide form.

2. A process according to the preceding claim, in which the quantity of nickel in said catalyst is in the range 0.01% to 50% by weight of the catalyst mass.

3. A process according to claim 1 ou 2, in which said additive is selected from glycine, aspartic acid, citric acid and tartaric acid.

4. A process according to claim 3, in which said additive is citric acid.

5. A process according to one of the preceding claims, in which the additive/nickel molar ratio in the solution from step a) is in the range 0.05 to 1.5.

6. A process according to claim 5 in which the additive/nickel molar ratio in the solution from step a) is in the range 0.1 to 1.5.

7. A process according to one of the preceding claims, in which drying step c) is carried out at a temperature in the range 20°C to 160°C for a period in the range 1 to 24 hours.

8. A process according to one of the preceding claims, in which calcining step d) is carried out at a temperature in the range 200°C to 800°C for a period in the range 1 to 6 hours.

9. A process according to one of the preceding claims, comprising at least one reduction treatment step f) carried out by contact with a reducing gas at a temperature of 100°C or more for a period or 2 hours or more.

10. A process according to one of claims 1 to 9, comprising a step e) for passivation of the catalyst carried out by contact with at least one sulphur-containing compound, after calcining step d) and before the reduction treatment step f).

11. A process according to claims 1 to 10, in which said polyunsaturated hydrocarbon feed is selected from a C2 steam cracking cut, a C3 steam cracking cut, a C4 steam cracking cut, a C5 steam cracking cut and steam cracked gasolines.

12. A process according to claims 1 to 11, in which said polyunsaturated hydrocarbon feed is a steam cracked gasoline.
